# EUROPEAN PATENT APPLICATION

(11) **EP 1 834 652 A1**
(43) Date of publication of application: **19.09.2007**
(21) Application number: 06711450.4
(22) Date of filing: 06.01.2006
(51) Int. Cl.: A61K 47/04, A61K 9/08, A61K 9/14, A61K 47/02, A61K 47/12, A61K 47/26, A61P 7/08

(54) **DIALYSIS PREPARATION**

(30) Priority: 07.01.2005 JP 2005002781
(71) Applicant: Ajinomoto Co., Inc., Tokyo 104-8315 (JP)
(72) Inventor: IWASHINA, Hirotoshi, ku, Shizuoka-shi, Shizuoka, 4240911 (JP); KAMIYA, Kiyonobu, -ku, Kawasaki-shi, Kanagawa, 2108681 (JP)
(74) Representative: Albrecht, Thomas
(86) International application number: PCT/JP2006/300051
(87) International publication number: WO 2006/073164

(57) **Abstract**

The present invention provides a pharmaceutical composition for dialysis wherein the decline of ionized calcium concentration in an acetate-free bicarbonate dialysate comprising no acetic acid and/or sodium acetate is controlled. The present invention further provides a dialysis agent A that comprises electrolytes, citric acid and/or citrate as pH adjuster, and/or glucose, for preparation of bicarbonate dialysates; the agent A **characterized by** being adjusted by citric acid and/or citrate so as to keep the electrolyte ionized calcium concentration not less than 1 mmol/L in a prepared bicarbonate dialysate. In a specific aspect of the present invention, sodium citrate is used as citrate, and the present invention provides a dialysate obtained from above.

## Description

### TECHNICAL FIELD

The present invention relates to a bicarbonate dialysate for hemodialysis, and in particular, relates to a bicarbonate dialysate that comprises no acetic acid and/or sodium acetate as pH adjuster and that comprises citric acid and/or citrate, in a preferred embodiment, sodium citrate as pH adjuster to inhibit the decline of electrolyte ionized calcium (Ca⁺⁺) concentration.

### BACKGROUND ART

One of the most general hemopurification therapeutic methods for Chronic Renal Failure (CRF) patients is hemodialysis (an artificial dialysis treatment). Besides the removal of wastes and water from blood, a purpose of hemodialysis is to correct the serum electrolyte concentration and the acid-base equilibrium.

Dialysates for hemodialysis are required to comprise a lot of alkalizer in order to neutralize CRF-induced metabolic acidosis. Thus, it is reasonable that bicarbonate be an optimal alkalizer for such dialysates. Therefore, bicarbonate dialysates have been used in hemodialysis treatments. However, there have been problems in bicarbonate dialysates such as; the instability because of the reaction between included bicarbonate ions and calcium irons as well as magnesium ions to form insoluble compounds (metal carbonate compounds like calcium carbonate and magnesium carbonate); and the difficulty in long-term storage for the bacteria-prone nature.

For the solution of the above, a method for alkali supplementation utilizing the conversion of acetic acid into bicarbonic acid by metabolism in the liver was established. Since this method was able to supply stable dialysates, acetate dialysis has become popular using sodium acetate as acetate for alkalization. As a result, it has become possible to supply enough bicarbonate ions because the method has enabled to set a high alkali concentration. On the other hand, however, other problems have appeared such as; acetic acid has a vasodilatory effect and a cardiac function depression effect, which can lower the blood pressure; acetic acid can deteriorate acetate-induced dialysis disequilibrium syndrome in the acetate intolerance patients whose metabolism of acetic acid is slow; and respiratory depression can be caused by the large loss of blood carbon dioxide (CO₂) during dialysis.

Later, given the increase of dialysis-requiring patients and the expansion of target patients having diabetic renal disease as primary disease, dialysis disequilibrium syndrome has come to be seen more frequently and severely. Further, more patients receiving ordinary dialysis have come to request asymptomatic dialysis that causes slight discomfort during dialysis. Since acetic acid was a presumable cause of the above symptoms, lately bicarbonate dialysis with sodium hydrogencarbonate as alkalizer has become dominant in stead of acetate dialysis using acetate.

Usually bicarbonate dialysis consists of two solutions of so-called "agent A" , which has electrolytes comprising calcium and magnesium ions; pH adjuster; and/or glucose; and so-called "agent B", which contains sodium bicarbonate to be bicarbonate ions; in order to inhibit the formation of insoluble metal carbonate compounds (such as calcium carbonate and magnesium carbonate) by the reaction between bicarbonate irons and calcium ions as well as magnesium ions. Although not so much as conventional acetate dialysates, bicarbonate dialysates still comprise acetic acid and sodium acetate as pH adjuster in the concentration of about 6-12 mEq/L to adjust the solution pH ranging 7 to 8.

Initially, adding acetic acid in the above concentration was thought unproblematic. Lately, however, involved in the prolongation of dialysis-receiving period, there have appeared such problems as hypotension during dialysis and clinical manifestations presumably induced by acetic acid, because primarily practically no acetic acid exists in a biological body (the blood concentration: not more than 0.1 mEq/L).

In addition, it has become recognized that the toxic action of acetic acid including acetic acid intolerance is stronger than ever thought; due to the development of dialyzer function and other factors, acetic acid has come to be loaded excessively, giving adverse effects on the cardiovascular system. Accordingly, there remained a need for the development of acetate-free bicarbonate dialysates. For the solution of this, the use of citric acid and/or sodium citrate as alkalizer was suggested. (See Patent Document 1.)

Citric acid and/or sodium citrate as alkalizer in bicarbonate dialysate solved above-mentioned clinical problems deriving from acetic acid. On the other hand, however, the chelating action of citric acid was not a negligible problem. Adding a lot of citrate acid and/or sodium citrate could lower the ionized calcium concentration by chelating during dialysis to cause hypocalcemia.
Especially in dialysis patients, the standard ionized calcium concentration is set to be 2.05-2.60 mEq/L (1.025-1.30 mmol/L). (See non-patent literature 1.) Therefore, it is important to maintain this concentration regardless whether before dialysis or after.

Normally, in healthy adults, it is required that the serum calcium concentration be 8.0-10.5 mg/dL, of which the ionized calcium concentration be not less than 1 mmol/L. When bicarbonate dialysates comprising citrate acid and/or sodium citrate as pH adjuster were used, it was observed that included citrate acid and/or sodium citrate formed chelates with calcium and, as a result, the ionized calcium concentration was lowered.

As a result of a comparative study of Ca metabolism in chronic hemodialysis patients receiving bicarbonate dialysates and acetate dialysates, it was reported that Ca⁺⁺ decreased more significantly when bicarbonate dialysates were used than acetate dialysates, and that the bone mineral density also decreased more significantly when bicarbonate dialysates were used than acetate dialysates. (See Non-Patent Document 2.) The above-mentioned decline of ionized calcium concentration causes hypocalcemia and is not preferable to Ca and born turnovers. Thus, as supplementation of Ca⁺⁺ in low-Ca bicarbonate dialysates, administration of calcium carbonate preparations and activated vitamin D preparations was suggested (See Non-Patent Document 3).

Adversely, however, administration of calcium carbonate preparations can cause hypercalcemia. From this point of view, it is possible to maintain the ionized calcium concentration by setting the Ca⁺⁺ concentration of bicarbonate dialysate and increasing the electrolyte density, for example the calcium chloride concentration, in order to supplement decreased ionized calcium due to the chelate formation with citric acid and/or sodium citrate included as pH adjuster. However, the dense concentration of calcium ions in dialysates is not preferable, because hypercalcemia can be caused, and in addition, it is complicated to control the calcium concentration of dialysate.
Accordingly, there is a need for development of acetate-free dialysates wherein an optimal calcium concentration as electrolyte for ordinary bicarbonate dialysates is maintained and the ionized calcium concentration is not lowered.

Patent Document 1: Japanese Patent Publication No. 2003-104869
Non-Patent Document 1: Fumitake Shimojo, Examination and Control of Dialysis Patients, p. 32, 1st ed.: Chugai Medical Co., June 21, 1999.
Non-Patent Document 2: Eiichi Chiba et al. , Journal of The Japanese Society for Dialysis Therapy, 21(6): 517-522, 1988.
Non-Patent Document 3: Fumiyoshi Nakayama et al., Artificial Organs, 18(3): 1220-1224, 1989.

### DISCLOSURE OF THE INVENTION

### PROBLEMS TO BE SOLVED BY THE INVENTION

Under the above circumstances, an object of the present invention is to provide a pharmaceutical composition for dialysis that keeps the ionized calcium concentration not less than 1 mmol/L in acetate-free bicarbonate dialysates comprising no acetic acid and/or sodium acetate.

The present inventors have made extensive research with a view to solving the above problems. As a result, they have newly found that above objects can be attained by; minimizing the quantity of citric acid and/or sodium citrate used as pH adjuster in an acetate-free bicarbonate dialysate with an optimal concentration of calcium as additive electrolytes; and adjusting such quantity so as to keep the ionized calcium concentration in the dialysate not less than 1 mmol/L; thus, completing the present invention.

### MEANS TO SOLVE THE INVENTION

That is, the present invention provides, in a basic aspect, a dialysate characterized in that the citric acid and/or citrate content thereof is adjusted so as to keep the electrolyte ionized calcium concentration not less than 1 mmol/L in a bicarbonate dialysate comprising citric acid and/or citrate as pH adjuster.

Further specifically, the present invention provides a dialysate characterized in that the electrolyte ionized calcium concentration is adjusted so as to be not less than 1 mmol/L by a combination of citric acid and/or sodium citrate as pH adjuster, the combination comprising; setting the citric acid concentration of a dialysate not more than 1.5 mEq/L; and combining therewith sodium citrate having the concentration within a range of 0.2 to 0.5 mEq/L.

The present invention also provides, in another aspect, a method of adjusting the electrolyte ionized calcium concentration, by citric acid and/or citrate, so as to be not less than 1.0 mmol/L in a bicarbonate dialysate that comprises no acetic acid and/or acetate and comprises citric acid and/or citrate as pH adjuster.

More specifically in this instance, the present invention provides a method wherein the electrolyte ionized calcium concentration is adjusted so as to be not less than 1 mmol/L by a combination of citric acid and/or sodium citrate as pH adjuster, the combination comprising; setting the citric acid concentration of a dialysate not more than 1.5 mEq/L; and combining therewith sodium citrate having the concentration within a range of 0.2 to 0.5 mEq/L.

Furthermore, the present invention also provides, in another aspect, a dialysis agent A for preparation of bicarbonate dialysates, the agent A comprising electrolytes, citric acid and/or citrate as pH adjuster, and/or glucose; the agent A characterized in that the electrolyte ionized calcium concentration is adjusted so as to be not less than 1 mmol/L in a bicarbonate dialysate after preparation by citric acid and/or citrate.

More specifically, the present invention provides a dialysis agent A wherein the ionized calcium concentration is adjusted so as to be not less than 1 mmol/L by; setting the citrate acid concentration not more than 1.5 mEq/L; and combining therewith sodium citrate having the concentration within a range of 0.2 to 0.5 mEq/L.

The present invention also provides, in another aspect, a method of hemodialysis wherein a bicarbonate dialysate is prepared to obtain the ionized calcium concentration thereof being not less than 1 mmol/L without comprising acetic acid and/or acetate.

More specifically, the prevent invention provides a method of hemodialysis wherein the ionized calcium concentration is adjusted by the above-mentioned ionized calcium concentration adjusting methods.

### MEANS TO SOLVE THE INVENTION

The present invention provides a bicarbonate dialysate wherein the ionized calcium concentration is adjusted so as to be not less than 1 mmol/L. Thus, the present invention provides an excellent bicarbonate dialysate that does not distress Ca and born turnovers.
Further, an advantage of the present invention is that it provides an agent A for bicarbonate dialysate preparation, wherein the ionized calcium concentration is adjusted so as to be not less than 1 mmol/L in a prepared dialysate by the combination of content of citric acid and/or sodium citrate, and therefore, it can provide a stable bicarbonate dialysate.

### BEST MODE FOR CARRYING OUT THE INVENTION

In a first aspect, a bicarbonate dialysate provided by the present invention is characterized by the use of citric acid and/or sodium citrate, which exist in a biological body also, as pH adjuster without comprising acetic acid and/or sodium acetate that was conventionally used in bicarbonate dialysate. Therefore, the present invention enables a preparation of so-called acetate-free bicarbonate dialysate.

In a second aspect, a bicarbonate dialysate provided by the present invention is characterized in that; by adjusting the content of citric acid and/or sodium citrate as pH adjuster, the ionized calcium concentration is adjusted so as to be not less than 1 mmol/L, and at the same time, an optimal calcium concentration of dialysate is maintained.

Such bicarbonate dialysate is a dialysate obtained by diluting and mixing two agents of; so-called agent A comprising electrolytes, citric acid and/or sodium citrate as pH adjuster, and/or glucose; and so-called agent B comprising sodium hydrogencarbonate, that is, bicarbonate ions as alkalizer.
An agent A provided by the present invention can flexibly comprise optional components as dialysate elements at requests. Also, an agent B can flexibly comprise optional dialysate elements in addition to sodium hydrogencarbonate.

Accordingly, a dialysis agent A provided by the present invention is a pharmaceutical composition for preparation of bicarbonate dialysates to be diluted and mixed with a sodium hydrogencarbonate solution (so-called agent B); the formulation of the agent A can be liquid or solid.

In the case of solid formulation; because of the possibility of reaction between glucose and citric acid, or other components as the case maybe, when all components are integrated in a single formulation; each reacting component can be stored separately. In an extremely preferable embodiment, a multi-chamber container that has multiple separated chambers in a bag is used since all components can be mixed without fail. For example, electrolytes and glucose can be stored separately in two different chambers; or electrolytes, glucose, and citric acid and/or sodium citrate as pH adjuster can be stored separately in three different chambers.

As mentioned above, the present invention provides a bicarbonate dialysate wherein the ionized calcium concentration is adjusted so as to be not less than 1 mmol/L, and at the same time, an optimal calcium concentration of dialysate is maintained. The present inventors have found that specifically such adjustment becomes feasible by controlling the added quantity of citric acid and/or sodium citrate as pH adjuster.

According to investigations by the present inventors, they have found that the ionized calcium concentration can be adjusted so as to be not less than 1 mmol/L by setting the dialysate citric acid concentration to be not more than 1.5 mEq/L and combing thereto sodium citrate having the concentration within a range of 0.2 to 0.5 mEq/L.

In a dialysate provided by the present invention, a pH is controlled within a range of 7 to 8. Usually the content of pH adjuster cannot be determined without reservation because the pH adjuster content differs depending on the content of alkalizer. However, the present inventors have found that the above-mentioned content of citric acid and/or sodium citrate can control a pH within the target range and that the ionized calcium concentration can be adjusted so as to be not less than 1 mmol/L at the same time.

Furthermore, a dialysate agent A provided by the present invention usually comprises electrolytes, pH adjuster, and/or glucose; and the electrolytes thereof can be, for example but not limited to, citrate such as sodium citrate, sodium chloride, potassium chloride, magnesium chloride, calcium chloride, sodium lactate, potassium lactate, calcium lactate.

Preferable electrolytes are sodium chloride, potassium chloride, calcium chloride and magnesium chloride.

In a preferable embodiment, respective dialysate components are proportioned in the following concentration in a bicarbonate dialysate when diluted and mixed properly.

- Na⁺: 120-150 mEq/L
- K⁺: 0-4 mEq/L
- Ca²⁺: 0-4 mEq/L
- Mg²⁺: 0-1.5 mEq/L
- Cl⁻: 55-150 mEq/L
- HCO₃⁻: 20-45 mEq/L
- Citrate³⁻: 1-2 mEq/L
- Glucose: 0-3.0 g/L

In a preferable embodiment, according to the present invention, citric acid and/or sodium citrate are used as pH adjuster. The content of citric acid and/or sodium citrate is such as can control a pH ranging about 7 to 8; minimize chelating with ionized calcium; and adjust the ionized calcium concentration so as to be not less than 1 mmol/L; in a prepared bicarbonate dialysate, that is, a solution obtained by properly mixing and diluting both agents A and B.

Conventionally an agent A for bicarbonate dialysates comprises acetic acid and/or sodium acetate as pH adjuster. However, an agent A provided by the present invention comprises citric acid and/or sodium citrate as pH adjuster without comprising acetate. Thus, an advantage of the present invention is that it provides a more physiological formulation because it is feasible to use sodium hydrogencarbonate only as alkalizer.

Another advantage of the present invention is that a precipitate-formation inhibiting effect can be expected because of the use of citric acid and/or sodium citrate. Conventionally, for example, so-called agent A comprising electrolytes, pH adjuster, and/or glucose, and so-called agent B comprising sodium hydrogencarbonate, that is, bicarbonate irons are mixed and diluted just before the use to prepare a bicarbonate dialysate; because bicarbonate ions react to calcium ions and magnesium ions to form insoluble metal carbonate compounds.

Generally it takes approximately 3 to 5 hours to practically complete a hemodialysis using a prepared bicarbonate dialysate. Therefore, a bicarbonate dialysate is required to be stable longer than the above time. The present investigators have found that a bicarbonate dialysate provided by the present invention was stable for a long time after preparation without precipitate detected. This stability derives from an effect of citric acid ions (citrate³⁻) in the dialysate originating from citric acid and/or sodium citrate used as pH adjuster. In this point also, the present invention has an excellent clinical advantage.

### EXAMPLES

By way of example, and not limitation, specifically detailed description of the invention will now be set forth.

### Example 1:

An agent A for bicarbonate dialysate preparation comprising electrolytes, citric acid and/or sodium citrate as pH adjuster, and glucose was prepared in the five formulations as follows.
With 214 .8 g of sodium chloride (NaCl) , 5 . 222 g of potassium chloride (KCl), 7.72 g of calcium chloride (CaCl₂ 2H₂O), 3.56 g of magnesium chloride (MgCl₂ 6H₂O) as electrolytes, 52 . 5 g of glucose, and citric acid (C₆H₈O₇ H₂O) and sodium citrate (C₆H₅Na₃O₇ 2H₂O) as pH adjuster in Table 1 were dissolved in water to make 1 L of agent A.
The content of citric acid and sodium citrate as pH adjuster was prepared so as to obtain the citric acid and sodium citrate concentration being the concentration in Table 1 in the mixed-and-diluted solutions with below-mentioned agent B.

**[Table 1]**

| Formulation | Concentration in the prepared dialysates | |
|---|---|---|
| | Citric acrid concentration | Sodium citrate concentration |
| 1 | 1.4 mEq/L | 0.5 mEq/L |
| 2 | 1.4 mEq/L | 0.45 mEq/L |
| 3 | 1.4 mEq/L | 0.4 mEq/L |
| 4 | 1.4 mEq/L | 0.3 mEq/L |
| 5 | 1.8 mEq/L | 0.6 mEq/L |

At the same time, 29.404 g of sodium hydrogencarbonate as alkalizer was dissolved in water to make 1 L (agent B stock solution).

### [Experiment Methods]

### Experiment No. 1: Ionized Calcium Concentration and pH of Dialysates Prepared by a Dialysate-Proportioning System

The agent A formulated according to the above five formulations and the agent B stock solution were proportioned in a single-patient dialysis system to prepare dialysates. The ionized calcium concentration and the pH in the prepared dialysates were measured with two blood-electrolyte analyzers of; "i-STAT" of i-STAT Corporation using a test cartridge CG8+type for ionized calcium measurement; and ABL77 (Radiometer Trading KK).
For comparison, dialysates were prepared and tested in the same method using AK-SOLITA(TM)-DL (liquid) and AK-SOLITA(TM)-FL (liquid), artificial-kidney dialysates comprising acetate acid and sodium acetate as pH adjuster, on the market.

### Experiment No. 2: Ionized Calcium Concentration and pH of Dialysates Prepared with Laboratory Glassware

For testing in the dialysates prepared with laboratory glassware, dialysates were prepared as follows. The above-mentioned agent B stock solution measured precisely in the quantity according to Table 2 was put in a 350 mL measuring flask; thereto 300 mL of water was added; further thereto 10 mL of agent A formulated according to the above formulations (Formulation 1 to 5) was added; and further thereto water was added to make just 350 mL.
Like Experiment No . 1, dialysates were prepared and tested in the same methods using AK-SOLITA(TM)-DL (liquid) and AK-SOLITA(TM)-FL (liquid).

**[Table 2]**

| Formulation | Concentration in the prepared dialysates | Agent B stock solution |
|---|---|---|
| 1 | citric acid 1.4 mEq/L citric acid 1.4 mEq/L + sodium citrate 0.5 mEq/L | 35 mL |
| 2 | citric acid 1.4 mEq/L + sodium citrate 0.45 mEq/L | 35 mL |
| 3 | citric acid 1.4 mEq/L + sodium citrate 0.4 mEq/L | 35 mL |
| 4 | citric acid 1.4 mEq/L + sodium citrate 0.3 mEq/L | 35 mL |
| ⁵ | citric acid 1.8 mEq/L + sodium citrate 0.6 mEq/L | 35 mL |
| AK-SOLITA-DL | - | 25 mL |
| AK-SOLITA-FL | - | 27.5 mL |

### Results of the above experiments are shown in Tables 3 and 4.

Table 3 shows the results in the dialysates prepared by a single-patient dialysis system. Table 4 shows the results in the dialysates prepared with laboratory glassware.

**[Table 3]**

| Formulation | i-STAT assay results | | ABL assay results | |
|---|---|---|---|---|
| | Ionized Ca concentration | PH | Ionized Ca concentration | pH |
| 1 | 1.01 | 7.77 | 1.11 | 7.64 |
| 2 | 1.03 | 7.79 | 1.13 | 7.63 |
| 3 | 1.03 | 7.78 | 1.13 | 7.62 |
| 4 | 1.05 | 7.78 | 1.15 | 7.62 |
| 5 | 0.93 | 7.67 | 1.05 | 7.54 |
| AK-SOLITA-DL | 1.35 | 7.28 | 1.48 | 7.28 |
| AK-SOLITA-FL | 1.14 | 7.34 | 1.26 | 7.32 |

**[Table 4]**

| Formulation | i-STAT assay results | | ABL assay results | |
|---|---|---|---|---|
| | Ionized Ca concentration | PH | Ionized Ca concentration | pH |
| 1 | 1.03 | 7.63 | 1.14 | 7.52 |
| 2 | 1.04 | 7.63 | 1.15 | 7.52 |
| 3 | 1.04 | 7.59 | 1.15 | 7.52 |
| 4 | 1.07 | 7.60 | 1.17 | 7.53 |
| 5 | 0.95 | 7.49 | 1.05 | 7.45 |
| AK-SOLITA-DL | 1.36 | 7.27 | 1.49 | 7.28 |
| AK-SOLITA-FL | 1.14 | 7.32 | 1.27 | 7.32 |

As the above results show, it is understandable that the ionized calcium concentration is adjusted so as to be not less than 1 mmol/L by; fixing the dialysate calcium concentration at a common concentration (3.0 mEq/L); setting the citric acid concentration as pH adjuster to be not more than 1.5 mEq/L; and combining thereto sodium citrate having the concentration within a range of 0.2 to 0.5 mEq/L.

### Example 2: Adjustment of Ionized Calcium Concentration by Increasing Calcium Concentration in Dialysate

The dialysates described in the Example 1 are examples of adjusting the ionized calcium concentration in dialysate by fixing the dialysate calcium concentration at a common concentration (3.0 mEq/L) and controlling the content of citric acid and sodium citrate as pH adjuster.
The ionized calcium concentration of dialysates can be adjusted by increasing the calcium concentration of dialysates, also.
In the Example 2, a dialysate was prepared with an agent A comprising the same electrolytes as the Formulation 4 in the Example 1, except for the calcium concentration of electrolytes changed to 3.5 mEq/L in dialysate; and agent B stock solution (35 mL).
According to the assay of the ionized calcium concentration and the pH of the prepared dialysate with blood-electrolyte analyzers (the two of i-STAT and ABL), the ionized calcium concentration was 1.15 mmol/L and the pH was 7.53 with i-STAT; and the ionized calcium concentration was 1.26 mmol/L and the pH was 7.46 with ABL.
From the above results, it is understandable that the ionized calcium concentration of dialysates can be adjusted by increasing the calcium concentration of dialysates, also.

### Example 3: Investigation of Ionized Calcium (Ca²⁺) Concentration in Clinical Trials

Variation of blood ionized calcium concentration (Ca²⁺) in the patients receiving dialysis using dialysates provided by the present invention was observed in clinical cases.
The Formulation 4 in the Example 1 was used for dialysates.

In the patients who received dialysis (three times a week) using dialysates of the present invention, the blood ionized-calcium concentration before dialysis (in the zero week, 0-week value; 4 weeks later, 4^{th}-week value; 7 weeks later, 7^{th}-week value; 8 weeks later, 8^{th}-week value; Table 5) and the blood ionized-calcium concentration after dialysis (in the 1^{st} week, 1^{st}-week value; 5 weeks later, 5^{th}-week value, 8 weeks later, 8^{th}-week value; Table 6) were assayed and observed.

### Results:

The assay results of ionized calcium concentration in mean, minimum and maximum are shown in Tables 5 and 6.

**[Table 5]**

| Ca²⁺ Concentration (mmol/L) before dialysis | 0-week | 4^{th}-week | 7^{th}-week | 8^{th}-week |
|---|---|---|---|---|
| Mean | 1.195 | 1.183 | 1.192 | 1.192 |
| ± S.D. | 0.134 | 0.132 | 0.149 | 0.149 |
| Maximum | 1.43 | 1.55 | 1.70 | 1.71 |
| Minimum | 0.85 | 0.80 | 0.83 | 0.73 |

**[Table 6]**

| Ca²⁺ Concentration (mmol/L) After dialysis | 1^{st}-week | 5^{th}-week | 8^{th}-week |
|---|---|---|---|
| Mean | 1.164 | 1.164 | 1.168 |
| ± S.D. | 0.102 | 0.092 | 0.100 |
| Maximum | 1.45 | 1.43 | 1.48 |
| Minimum | 0.80 | 0.92 | 0.83 |

As shown in the above tables, it is understandable that the blood ionized-calcium concentration in the dialysis patients who received dialysis using dialysates provided by the present invention was kept around the standard range of 2.05-2.60 mEq/L (See non-patent literature 1).

Further, the blood calcium concentration of the dialysis patients was kept within the standard rang in the 8-week continuous use.

As the above results show, the blood ionized-calcium concentration was kept within the standard range when acetate-free dialysis agents provided by the present invention were used. Therefore, the availability of dialysates provided by the present invention is well understandable.

### INDUSTRIAL APPLICABILITY

As described above, the present invention provides a pharmaceutical composition for dialysates that prevents the decline of ionized-calcium concentration in dialysates.
Further, other advantages of the present invention are that a bicarbonate dialysate agent A provided by the present invention is adjusted specifically by combining the content of citric acid and sodium citrate so as to keep the ionized-calcium concentration of dialysate not less than 1 mmol/L after preparation, and a stable bicarbonate dialysate can be provided.
Thus, a medical value of the present invention is great in terms of providing an excellent bicarbonate dialysate that does not distress Ca and born turnovers

## Claims

1. A dialysate **characterized in that** the citric acid and/or citrate content thereof is adjusted so as to keep the electrolyte ionized calcium concentration not less than 1 mmol/L in a bicarbonate dialysate comprising citric acid and/or citrate as pH adjuster.

2. The dialysate of claim 1 **characterized in that** the electrolyte ionized calcium concentration is adjusted so as to be not less than 1 mmol/L by a combination of citric acid and/or sodium citrate as pH adjuster, the combination comprising; setting the citric acid concentration of a dialysate not more than 1.5 mEq/L; and combining therewith sodium citrate having the concentration within a range of 0.2 to 0.5 mEq/L.

3. A method of adjusting the electrolyte ionized calcium concentration, by citric acid and/or citrate, so as to be not less than 1. 0 mmol/L in a bicarbonate dialysate that comprises no acetic acid and/or acetate and comprises citric acid and/or citrate as pH adjuster.

4. The method of claim 3 wherein the electrolyte ionized calcium concentration is adjusted so as to be not less than 1 mmol/L by a combination of citric acid and/or sodium citrate as pH adjuster, the combination comprising; setting the citric acid concentration of a dialysate not more than 1.5 mEq/L; and combining therewith sodium citrate having the concentration within a range of 0.2 to 0.5 mEq/L.

5. A dialysis agent A for preparation of bicarbonate dialysates, the agent A comprising electrolytes, citric acid and/or citrate as pH adjuster, and/or glucose; the agent A **characterized in that** the electrolyte ionized calcium concentration is adjusted so as to be not less than 1 mmol/L in a bicarbonate dialysate after preparation by citric acid and/or citrate.

6. The dialysis agent A of claim 5 wherein the electrolyte ionized calcium concentration is adjusted so as to be not less than 1 mmol/L by; setting the citrate acid concentration not more than 1.5 mEq/L; and combining therewith sodium citrate having the concentration within a range of 0.2 to 0.5 mEq/L.

7. The dialysis agent A of claim 6 **characterized in that** the formulation thereof is powder.

8. The dialysis agent A of claim 6 **characterized in that** the formulation thereof is liquid.

9. A method of hemodialysis **characterized in that** a bicarbonate dialysate is prepared to obtain the ionized calcium concentration thereof being not less than 1 mmol/L without comprising acetic acid and/or acetate.

10. The method of hemodialysis of claim 9 wherein the ionized calcium concentration is adjusted by the methods of claim 3 or claim 4.
